# FASCICULE DE BREVET EUROPEEN

(11) **EP 1 093 801 B1**
(45) Date de publication et mention de la délivrance du brevet: **02.06.2004**
(21) Numéro de dépôt: 00402371.9
(22) Date de dépôt: 28.08.2000
(51) Int. Cl.: A61K 7/48, A61K 7/02, A61K 7/06, A61K 7/50

(54) **Emulsion eau-dans-huile et ses utilisations notamment dans le domaine cosmétique**
W/O-Emulsion und ihre Verwendung insbesondere in der Kosmetik
W/O-emulsion and its use especially in cosmetics

(30) Priorité: 21.10.1999 FR 9913146
(43) Date de publication de la demande: 25.04.2001
(73) Titulaire: L'OREAL, 75008 Paris (FR)
(72) Inventeur: Lorant, Raluca, 94320 Thiais (FR)
(74) Mandataire: Rasson, Catherine

(56) Documents cités:
- EP-A- 0 531 943
- FR-A- 2 669 345
- US-A- 5 639 797

## Description

La présente invention se rapporte à une émulsion eau-dans-huile comprenant un alkylpolyglycoside ayant un HLB inférieur à 7 et au moins une huile volatile, et à ses utilisations notamment dans le domaine cosmétique. Elle peut être destinée en particulier au soin de la peau, des lèvres, des ongles et des cheveux, au démaquillage et/ou au nettoyage de la peau, et/ou au maquillage de la peau et/ou des lèvres. Elle peut être plus particulièrement utilisée pour le traitement des peaux et/ou lèvres sèches et/ou peaux sensibles.

Dans le domaine cosmétique, il est courant d'utiliser des crèmes constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour le soin et la réparation des peaux sèches et déshydratées auxquelles elles apportent confort et protection grâce à la barrière lipidique qu'elles forment sur la peau.

Toutefois, malgré leur grande efficacité, les émulsions E/H sont minoritaires parmi les formes galéniques utilisées dans le domaine cosmétique car elles posent deux problèmes majeurs. Tout d'abord, ces émulsions ont l'inconvénient de manquer généralement d'agrément cosmétique, c'est-à-dire qu'elles sont grasses, lourdes, collantes et manquent de fraîcheur du fait de la phase externe huileuse. Elles sont en général difficiles à appliquer sur la peau, pénètrent difficilement et laissent sur la peau un film rémanent brillant et souvent collant.

Par ailleurs, les émulsions E/H présentent des problèmes de stabilité et il est souvent nécessaire, pour les stabiliser, d'utiliser un fort taux d'émulsionnants et/ou d'introduire une certaine quantité de facteurs de consistance, tels que les cires. Toutefois, ces ingrédients contribuent à accentuer les défauts cosmétiques (effet poisseux et gras) des émulsions E/H et il en résulte l'obtention de compositions souvent compactes et lourdes. De plus, si on augmente fortement la teneur en émulsionnant de ces émulsions pour remédier à leur instabilité, les émulsions obtenues peuvent se montrer irritantes vis-à-vis de certains types de peau, notamment les peaux sensibles.

Il subsiste donc le besoin d'une émulsion eau-dans-huile stable ne présentant pas les inconvénients rencontrés avec celles connues jusqu'à présent.

L'émulsion selon l'invention permet de pallier les problèmes mentionnés précédemment. En effet, la demanderesse a trouvé de manière surprenante qu'il était possible d'obtenir une émulsion eau-dans-huile ayant de bonnes propriétés cosmétiques (toucher léger, frais et riche à la fois) et une bonne stabilité en utilisant un alkylpolyglycoside particulier comme émulsionnant, et des huiles volatiles.

Certes, il est connu d'utiliser des alkylpolyglycosides (APG) comme émulsionnants, mais ils ont généralement un HLB (Hydrophilic Lipophilic balance) supérieur à 8 et sont généralement utilisés pour la préparation d'émulsions H/E. Pour obtenir des émulsions E/H, il est généralement nécessaire de les associer à un autre APG ou à un autre tensioactif. Ainsi, le document US-A-5,639,797 décrit une émulsion E/H contenant un APG hydrophile tel que le décylglucoside, c'est-à-dire ayant un HLB supérieur à 8, qu'il faut associer à un tensioactif E/H pour obtenir l'émulsion voulue. Par ailleurs, il est souvent nécessaire de mettre une quantité totale de tensioactifs importante pour obtenir une émulsion E/H stable. La présente invention permet d'éviter ces inconvénients.

Par ailleurs, le document FR-A-2,669,345 décrit des compositions lavantes et moussantes contenant un milieu aqueux, des silicones insolubles dans le milieu et des alkylpolyglycosides.

Aussi, la présente invention a pour objet une émulsion eau-dans-huile comportant une phase aqueuse dispersée dans une phase huileuse, caractérisée en ce qu'elle comprend (i) un alkylpolyglycoside ayant un HLB inférieur à 7, et (ii) au moins une huile volatile.

Les émulsions obtenues selon l'invention s'étalent facilement, sont absorbées rapidement et complètement dans la peau. En même temps qu'un toucher riche et nutritif, elles apportent une sensation de fraîcheur surprenante. Après application du produit, la peau reste douce et mate. Par ailleurs, les émulsions de l'invention peuvent avoir une texture crème ou être fluides tout en ayant une bonne stabilité. Les émulsions fluides ont généralement une viscosité allant d'environ 0,2 à 3 Pa.s (2 à 30 poises) et de préférence de 0,6 à 2 Pa.s (6 à 20 poises), cette viscosité étant mesurée à environ 25°C à l'aide du viscosimètre "Rhéomat Metler" équipé d'un mobile 2 (pour les viscosités inférieures à 7 poises) ou d'un mobile 3 (pour les viscosités supérieures à 7 poises).

Les alkylpolyglycosides utilisés selon l'invention ont un HLB inférieur à 7 et de préférence inférieur ou égal à 5, tandis que les alkylpolyglycosides habituellement utilisés dans les compositions cosmétiques ont généralement un HLB supérieur à 10 et sont soit des détergents (HLB généralement supérieur à 13) soit des émulsionnants d'émulsion huile-dans-eau (HLB de 10 à 17 environ). Le HLB peut être calculé notamment par la méthode de Davies ou la méthode de Griffin.

Les alkylpolyglycosides utilisés selon la présente invention peuvent être plus particulièrement représentés par la formule générale (I) suivante :

R-O-(G)ₓ (I)

dans laquelle R représente un radical alkyle ramifié et/ou insaturé, comportant de 14 à 24 atomes de carbone, G représente un sucre réduit comportant de 5 à 6 atomes de carbone, et x désigne une valeur allant de 1 à 10 et de préférence de 1 à 4.

G désigne notamment le glucose, le fructose ou le galactose.

Le radical alkyle insaturé (radical alkylène) peut comprendre une ou plusieurs insaturations éthyléniques, et en particulier une ou deux insaturations éthylèniques.

Les alkylpolyglycosides préférés selon la présente invention sont des composés de formule (I) dans laquelle R représente plus particulièrement un radical oléyle (radical insaturé en C18) ou isostéaryle (radical saturé en C18), G désigne le glucose, x est une valeur allant de 1 à 2.

L'alkylpolyglycoside utilisé dans l'émulsion de l'invention est de préférence choisi dans le groupe comprenant l'isostéaryl-glucoside, l'oléyl-glucoside et leurs mélanges.

Selon un mode particulier de réalisation de l'invention, l'émulsion selon l'invention peut contenir, outre l'alkylpolyglycoside indiqué ci-dessus, un co-émulsionnant. De manière avantageuse, le co-émulsionnant peut être choisi parmi les alcools gras et notamment ceux ayant la même chaîne grasse que celle de l'alkylpolyglucoside, c'est-à-dire comportant de 14 à 24 atomes de carbone et ayant une chaîne ramifiée et/ou insaturée, et par exemple l'alcool isostéarylique quand l'alkylpolyglycoside est l'isostéaryl-glucoside, et l'alcool oléylique quand l'alkylpolyglycoside est l'oleyl-glucoside.

Selon un mode préféré de réalisation de l'invention, on utilise dans l'émulsion de la présente invention, le mélange de l'alkylpolyglycoside tel que défini ci-dessus avec l'alcool gras correspondant sous forme d'une composition autoémulsionnante, comme décrit par exemple dans le document WO-A-92/06778.

L'émulsion selon l'invention peut contenir une quantité d'alkylpolyglycoside(s) allant par exemple de 0,1 à 10 %, et de préférence de 0,2 à 5 % en poids de matière active par rapport au poids total de la composition. Quand il est en mélange avec l'alcool gras correspondant, ce dernier est présent en une quantité allant généralement de 60 à 90 % en poids et de préférence de 70 à 87 % en poids par rapport au poids du mélange d'alkylpolyglycoside et d'alcool gras.

La phase huileuse de l'émulsion selon l'invention contient au moins une huile volatile. Par huile volatile, on entend dans la présente description toute huile susceptible de s'évaporer au contact de la peau et/ou de faible point éclair, c'est-à-dire ayant un point éclair inférieur à environ 100°C, et notamment de point éclair compris entre 30 et 85°C, c'est-à-dire une huile dont le point éclair est suffisamment élevé pour permettre l'utilisation de ces huiles en formulation et suffisamment bas pour obtenir l'évanescence souhaitée sur la peau.

Comme huiles volatiles, on peut citer par exemple les huiles hydrocarbonées à chaîne ramifiée et les huiles de silicone volatiles, linéaires ou cycliques, telles que les cyclométhicones comme la cyclotétradiméthylsiloxane (point éclair 55°C), la cyclopentadiméthylsiloxane (point éclair environ 77°C), la cyclohexadiméthylsiloxane (point éclair 76°C), le méthylhexyldirnéthylsiloxane (point éclair 79°C). Ces huiles peuvent être utilisées seules ou en mélange.

Les huiles hydrocarbonées à chaîne ramifiée utilisables comme huiles volatiles dans l'émulsion de l'invention comportent de préférence de 6 à 20 et mieux de 6 à 18 atomes de carbone et peuvent être choisies par exemple dans le groupe comprenant l'isohexadécane, l'isododécane, les isoparaffines et leurs mélanges.

La phase huileuse peut comprendre en outre tous les corps gras classiquement utilisés dans le domaine cosmétique. Les corps gras peuvent être notamment choisis parmi les huiles autres que celles indiquées ci-dessus, les acides gras, les alcools gras et les cires.

Parmi les huiles utilisables dans l'émulsion de l'invention, on peut citer par exemple les huiles végétales telles que l'huile de noyaux d'abricot et l'huile de soja ; les huiles minérales comme l'huile de vaseline ; les huiles de synthèse ; les huiles de silicone non volatiles et les huiles fluorées. Comme autres corps gras, on peut citer par exemple la cire d'abeille et la vaseline.

Quand l'émulsion est utilisée comme produit de démaquillage de la peau et/ou des yeux, elle contient plus particulièrement des huiles démaquillantes et notamment celles choisies parmi les esters d'acide gras comportant au moins 12 atomes de carbone. Ces esters sont de préférence obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou di-esters. Les huiles démaquillantes peuvent être notamment choisies dans le groupe comprenant le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

La quantité de phase huileuse dans l'émulsion de l'invention peut aller par exemple de 5 à 50 % et de préférence de 10 à 40 % en poids par rapport au poids total de l'émulsion. La ou les huiles volatiles peuvent représenter tout ou une partie de cette phase huileuse, et elles représentent de préférence de 30 à 95 % en poids par rapport au poids total de la phase huileuse.

La phase aqueuse de l'émulsion peut représenter par exemple de 50 à 95 % et de préférence de 60 à 90 % en poids par rapport au poids total de l'émulsion.

L'émulsion de l'invention peut constituer notamment une composition cosmétique ou dermatologique, destinée à une application sur la peau, les cheveux et/ou les muqueuses. Elle contient alors avantageusement un milieu physiologiquement acceptable, c'est-à-dire compatible avec la peau, les muqueuses (lèvres) et/ou les cheveux.

De façon connue, l'émulsion de l'invention peut contenir également des adjuvants habituels dans le domaine cosmétique, tels que les actifs, les conservateurs, les antioxydants, les agents complexants, les solvants, les parfums, les charges, les filtres, les bactéricides, les absorbeurs d'odeur, les matières colorantes (pigments ou colorants solubles) et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Selon un mode particulier de réalisation de l'invention, l'émulsion comprend au moins une charge dont l'incorporation permet d'améliorer encore la stabilité de l'émulsion ainsi que son effet matifiant sur la peau. Comme charges qui peuvent être utilisées dans l'émulsion de l'invention, on peut citer par exemple, outre les pigments, les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges.

De manière préférée, on utilise comme charge les microsphères EXPANCEL, qui sont des particules de terpolymère expansé de chlorure de vinylidène, d'acrylonitrile et de méthacrylate, et notamment celles vendues sous les références 551 ayant une granulométrie allant d'environ 10 à 100 µm (551 DE 12, 551 DE 20, 551 DE 50, 551 DE 80) et celles vendues sous la référence EL 23 (granulométrie d'environ 18 µm).

Dans le cas des produits de maquillage et notamment des fonds de teint, la charge peut être constituée par des pigments.

Lorsque l'émulsion de l'invention contient des charges, la quantité de charge(s) peut aller par exemple de 0,01 à 15 % et de préférence de 0,1 à 5 % en poids par rapport au poids total de l'émulsion.

L'émulsion selon l'invention peut en outre contenir un ou plusieurs sels, et notamment un sel de magnésium tel que le sulfate de magnésium. La quantité de sel(s) peut aller par exemple de 0,1 à 5 % et de préférence de 0,5 à 1 % en poids par rapport au poids total de l'émulsion.

L'émulsion selon l'invention trouve son application dans un grand nombre de traitements, notamment cosmétiques, de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres. Elle peut être destinée aussi au traitement des peaux sèches et/ou des lèvres sèches.

L'émulsion selon l'invention peut par exemple être utilisée comme produit de soin, de démaquillage et/ou de nettoyage pour le visage sous forme de crèmes ou de laits ou comme produits de maquillage (peau et lèvres), et par exemple fonds de teint, par incorporation de charges ou de colorants.

Aussi, l'invention a encore pour objet l'utilisation cosmétique de l'émulsion telle que définie ci-dessus pour le traitement, la protection, le soin, le démaquillage et/ou le nettoyage de la peau, des lèvres et/ou des cheveux, et/ou pour le maquillage de la peau et/ou des lèvres.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une émulsion telle que définie ci-dessus.

L'invention a aussi pour objet l'utilisation de l'émulsion telle que définie ci-dessus pour la fabrication d'une composition destinée au soin des peaux sèches et/ou des lèvres sèches et/ou des peaux sensibles.

Les exemples ci-après d'émulsions selon l'invention sont donnés à titre d'illustration. Les quantités y sont données en % en poids, sauf mention contraire.

### Exemple 1 : Fluide hydratant pour tous types de peaux

| *Phase huileuse* : | |
|---|---|
| Isostéaryl-glucoside/alcool isostéarylique (15/85) | 5 % |
| (soit 0,75 % de matière active d'alkyl-polyglycoside) | |
| Isohexadécane | 15 % |
| Cyclohexaméthicone | 10 % |

| *Charge :* | |
|---|---|
| Expancel 551 | 1 % |

| *Phase aqueuse* : | |
|---|---|
| Glycérine | 5 % |
| Sulfate de magnésium | 0,5 % |
| Conservateurs | 0,4 % |
| Eau | qsp 100 % |

Mode opératoire : on disperse la charge dans la phase huileuse, puis on émulsionne en dispersant sous agitation vigoureuse la phase aqueuse dans le mélange obtenu.

On obtient ainsi un fluide très doux à l'application, riche et frais à la fois, non gras, pénétrant rapidement dans la peau qu'il laisse douce, mate et souple.

### Exemple 2 : Crème de soin nourrissante pour peaux sèches et sensibles

| *Phase huileuse :* | |
|---|---|
| Isostéaryl-glucoside/alcool isostéarylique (15/85) | 3 % |
| (soit 0,45 % de matière active d'alkyl-polyglycoside) | |
| Huile de noyaux d'abricot | 8 % |
| Cyclohexaméthicone | 8 % |
| Vaseline | 1 % |
| Cire d'abeille | 1 % |

| *Phase aqueuse :* | |
|---|---|
| Glycérine | 5% |
| Sulfate de magnésium | 0,5 % |
| Conservateurs | 0,4 % |
| Eau | qsp 100 % |

Mode opératoire : on chauffe séparément à 75°C les phases huileuse et aqueuse, puis on émulsionne en dispersant sous agitation vigoureuse la phase aqueuse dans la phase huileuse.

On obtient ainsi une crème onctueuse, douce et riche à l'application. Elle pénètre facilement en apportant immédiatement un effet nutritif apaisant.

### Exemple 3 : Lait démaquillant pour peaux sèches

| *Phase huileuse :* | |
|---|---|
| Oleyl-glucoside/alcool oléylique (16,5/83,5) | 3 % |
| (soit 0,495 % de matière active d'alkyl-polyglycoside) | |
| Huile de vaseline | 10 % |
| Cyclopentaméthicone | 10 % |
| Palmitate d'octyle | 10 % |

| *Charge :* | |
|---|---|
| Expancel 551 | 0,5 % |

| *Phase aqueuse :* | |
|---|---|
| Glycérine | 5 % |
| Sulfate de magnésium | 0,5 % |
| Conservateurs | 0,4 % |
| Eau | qsp 100 % |

Mode opératoire : on disperse la charge dans la phase huileuse, puis on émulsionne en dispersant sous agitation vigoureuse la phase aqueuse dans le mélange obtenu.

On obtient ainsi un lait particulièrement agréable à l'utilisation et présentant des très bonnes propriétés démaquillantes. Très onctueux à l'application, il élimine en douceur le maquillage et les impuretés sans agresser ou irriter la peau. Après élimination la peau reste douce, mate et fraîche.

### Exemple 4 Fond de teint pour peaux sèches

| *Phase huileuse :* | |
|---|---|
| Isostéaryl-glucoside/alcool isostéarylique (15/85) | 3 % |
| (soit 0,45 % de matière active d'alkyl-polyglycoside) | |
| Huile de soja | 2 % |
| Cyclohexaméthicone | 18 % |

| *Charge :* | |
|---|---|
| Pigments bruns, rouges, jaunes | 5 % |

| *Phase aqueuse :* | |
|---|---|
| Glycérine | 5 % |
| Sulfate de magnésium | 0,5 % |
| Conservateurs | 0,4 % |
| Eau | qsp 100 % |

Mode opératoire : on disperse les charges dans la phase huileuse, puis on émulsionne en dispersant sous agitation vigoureuse la phase aqueuse dans le mélange obtenue.

On obtient ainsi un fond de teint de texture souple, très doux à l'application, facile à étaler. Il donne un résultat de maquillage homogène, mat et naturel.

## Revendications

1. Emulsion eau-dans-huile comportant une phase aqueuse dispersée dans une phase huileuse, **caractérisée en ce qu'**elle comprend (i) un alkylpolyglycoside ayant un HLB inférieur à 7, et (ii) au moins une huile volatile.

2. Emulsion selon la revendication 1, **caractérisée en ce que** l'alkylpolyglycoside est représenté par la formule (I) :
R-O-(G)ₓ (I)
dans laquelle R représente un radical alkyle ramifié et/ou insaturé, comportant de 14 à 24 atomes de carbone, G représente un sucre réduit comportant de 5 à 6 atomes de carbone, et x désigne une valeur allant de 1 à 10.

3. Emulsion selon la revendication précédente **caractérisée en ce que**, dans la formule (I), R représente un radical oléyle ou isostéaryle, G désigne le glucose et x est une valeur allant de 1 à 2.

4. Emulsion selon l'une quelconque des revendications précédentes, **caractérisée en ce que** l'alkylpolyglycoside est choisi dans le groupe comprenant l'isostéaryl-glucoside, l'oléyl-glucoside et leurs mélanges.

5. Emulsion selon l'une quelconque des revendications précédentes, **caractérisée en ce que** la quantité d'alkylpolyglycoside(s) va de 0,1 à 10 % en poids de matière active par rapport au poids total de l'émulsion.

6. Emulsion selon l'une quelconque des revendications précédentes, **caractérisée en ce que** l'alkylpolyglycoside se présente en mélange avec un alcool gras comportant de 14 à 24 atomes de carbone et ayant une chaîne ramifiée et/ou insaturée.

7. Emulsion selon la revendication précédente, **caractérisée en ce que** la quantité d'alcool gras va de 60 à 90 % en poids par rapport au poids du mélange d'alkylpolyglycoside et d'alcool gras.

8. Emulsion selon l'une quelconque des revendications précédentes, **caractérisée en ce que** l'huile volatile est choisie parmi les huiles hydrocarbonées à chaîne ramifiée, les huiles de silicone volatiles et leurs mélanges.

9. Emulsion selon l'une quelconque des revendications précédentes, **caractérisée en ce que** l'huile volatile est choisie parmi l'isohexadécane, l'isododécane, les isoparaffines, les cyclométhicones et leurs mélanges.

10. Emulsion selon l'une quelconque des revendications précédentes, **caractérisée en ce que** la quantité de phase huileuse va de 5 à 50 % en poids par rapport au poids total de l'émulsion.

11. Emulsion selon l'une quelconque des revendications précédentes, **caractérisée en ce qu'**elle comprend au moins une charge.

12. Emulsion selon la revendication précédente, **caractérisée en ce que** la quantité de charge(s) va de 0,01 à 15 % en poids par rapport au poids total de l'émulsion.

13. Emulsion selon l'une quelconque des revendications précédentes, **caractérisée en ce qu'**elle constitue une composition cosmétique.

14. Utilisation cosmétique de l'émulsion selon l'une quelconque des revendications précédentes pour le traitement, la protection, le soin, le démaquillage et/ou le nettoyage de la peau, des lèvres et/ou les cheveux, et/ou pour le maquillage de la peau et/ou des lèvres.

15. Procédé de traitement cosmétique de la peau, des cheveux, et/ou des lèvres, **caractérisé par le fait qu'**on applique sur la peau, les cheveux et/ou les lèvres, une émulsion selon l'une quelconque des revendications 1 à 13.

16. Utilisation de l'émulsion selon l'une quelconque des revendications 1 à 13 pour la fabrication d'une composition destinée au soin des peaux sèches et/ou des lèvres sèches et/ou des peaux sensibles.

## Patentansprüche

1. Waaser-in-Öl-Emulsion, die eine in einer Ölphase dispergierte wässerige Phase aufweist, **dadurch gekennzeichnet, dass** sie (i) ein Alkylpolyglykosid, das einen HLB-Wert von kleiner 7 aufweist, und (ii) mindestens ein flüchtiges Öl enthält.

2. Emulsion nach Anspruch 1, **dadurch gekennzeichnet, dass** das Alkylpolyglykosid der Formel (I) entspricht:
R-O-(G)ₓ (I),
worin bedeuten:
- R eine verzweigte und/oder ungesättigte Alkylgruppe, die 14 bis 24 Kohlenstoffatome aufweist,
- G einen reduzierten Zucker, der 5 bis 6 Kohlenstoffatome aufweist, und
- x einen Wert von 1 bis 10.

3. Emulsion nach dem vorhergehenden Anspruch, **dadurch gekennzeichnet, dass** in der Formel (I) R eine Oleylgruppe oder eine Isostearylgruppe, G Glucose und x einen Wert von 1 bis 2 bedeuten.

4. Emulsion nach einem der vorhergehenden Ansprüche, **dadurch gekennzeichnet, dass** das Alkylpolyglykosid unter Isostearylglucosid, Oleylglucosid und den Gemischen dieser Verbindungen ausgewählt ist.

5. Emulsion nach einem der vorhergehenden Ansprüche, **dadurch gekennzeichnet, dass** der Mengenanteil des Alkylpolyglykosids (der Alkylpolyglykoside) im Bereich von 0,1 bis 10 Gew.-% Wirkstoff, bezogen auf das Gesamtgewicht der Emulsion, liegt.

6. Emulsion nach einem der vorhergehenden Ansprüche, **dadurch gekennzeichnet, dass** das Alkylpolyglykosid im Gemisch mit einem Fettalkohol vorliegt, der 14 bis 24 Kohlenstoffatome aufweist und eine verzweigte und/oder ungesättigte Kette hat.

7. Emulsion nach dem vorhergehenden Anspruch, **dadurch gekennzeichnet, dass** der Mengenanteil des Fettalkohols im Bereich von 60 bis 90 Gew.-%, bezogen auf das Gewicht des Gemisches von Alkylpolyglykosid und Fettalkohol, liegt.

8. Emulsion nach einem der vorhergehenden Ansprüche, **dadurch gekennzeichnet, dass** das flüchtige Öl unter Kohlenwasserstoffölen mit verzweigter Kette, flüchtigen Siliconölen und den Gemischen dieser Verbindungen ausgewählt ist.

9. Emulsion nach einem der vorhergehenden Ansprüche, **dadurch gekennzeichnet, dass** das flüchtige Öl unter Isohexadecan, Isododecan, Isoparaffinen, Cyclomethiconen und den Gemischen dieser Verbindungen ausgewählt ist.

10. Emulsion nach einem der vorhergehenden Ansprüche, **dadurch gekennzeichnet, dass** der Mengenanteil der Ölphase im Bereich von 5 bis 50 Gew.-%, bezogen auf das Gesamtgewicht der Emulsion, liegt.

11. Emulsion nach einem der vorhergehenden Ansprüche, **dadurch gekennzeichnet, dass** sie mindestens einen Füllstoff enthält.

12. Emulsion nach dem vorhergehenden Anspruch, **dadurch gekennzeichnet, dass** der Mengenanteil des Füllstoffs (der Füllstoffe) im Bereich von 0,01 bis 15 Gew.-%, bezogen auf das Gesamtgewicht der Emulsion, liegt.

13. Emulsion nach einem der vorhergehenden Ansprüche, **dadurch gekennzeichnet, dass** es sich um eine kosmetische Zusammensetzung handelt.

14. Kosmetische Verwendung der Emulsion nach einem der vorhergehenden Ansprüche zur Behandlung, zum Schutz, zur Pflege, zum Abschminken und/oder zum Reinigen der Haut, der Lippen und/oder der Haare und/oder zum Schminken der Haut und/oder der Lippen.

15. Verfahren zur kosmetischen Behandlung der Haut, der Haare und/oder der Lippen, **dadurch gekennzeichnet, dass** eine Emulsion nach einem der Ansprüche 1 bis 13 auf die Haut, die Haare und/oder die Lippen aufgebracht wird.

16. Verwendung der Emulsion nach einem der Ansprüche 1 bis 13 zur Herstellung einer Zusammensetzung, die für die Pflege trockener Haut und/oder trockener Lippen und/oder empfindlicher Haut vorgesehen ist.

## Claims

1. Water-in-oil emulsion comprising an aqueous phase dispersed in an oily phase, **characterized in that** it comprises (i) an alkylpolyglycoside having an HLB of less than 7, and (ii) at least one volatile oil.

2. Emulsion according to Claim 1, **characterized in that** the alkylpolyglycoside is represented by formula (I) :
R-O-(G)ₓ (I)
in which R represents a branched and/or unsaturated alkyl radical containing from 14 to 24 carbon atoms, G represents a reduced sugar containing 5 or 6 carbon atoms and x denotes a value ranging from 1 to 10.

3. Emulsion according to the preceding claim, **characterized in that**, in formula (I), R represents an oleyl or isostearyl radical, G denotes glucose and x is a value ranging from 1 to 2.

4. Emulsion according to any one of the preceding claims, **characterized in that** the alkylpolyglycoside is chosen from the group comprising isostearylglucoside and oleylglucoside, and mixtures thereof.

5. Emulsion according to any one of the preceding claims, **characterized in that** the amount of alkylpolyglycoside(s) ranges from 0.1% to 10% by weight of active material relative to the total weight of the emulsion.

6. Emulsion according to any one of the preceding claims, **characterized in that** the alkylpolyglycoside is present as a mixture with a fatty alcohol containing from 14 to 24 carbon atoms and having a branched and/or unsaturated chain.

7. Emulsion according to the preceding claim, **characterized in that** the amount of fatty alcohol ranges from 60% to 90% by weight relative to the weight of the mixture of alkylpolyglycoside and fatty alcohol.

8. Emulsion according to any one of the preceding claims, **characterized in that** the volatile oil is chosen from branched-chain hydrocarbon-based oils and volatile silicone oils, and mixtures thereof.

9. Emulsion according to any one of the preceding claims, **characterized in that** the volatile oil is chosen from isohexadecane, isododecane, isoparaffins and cyclomethicones, and mixtures thereof.

10. Emulsion according to any one of the preceding claims, **characterized in that** the amount of oily phase ranges from 5% to 50% by weight relative to the total weight of the emulsion.

11. Emulsion according to any one of the preceding claims, **characterized in that** it comprises at least one filler.

12. Emulsion according to the preceding claim, **characterized in that** the amount of filler(s) ranges from 0.01% to 15% by weight relative to the total weight of the emulsion.

13. Emulsion according to any one of the preceding claims, **characterized in that** it constitutes a cosmetic composition.

14. Cosmetic use of the emulsion according to any one of the preceding claims, for treating, protecting, caring for, removing make-up from and/or cleansing the skin, the lips and/or the hair, and/or for making up the skin and/or the lips.

15. Cosmetic treatment process for the skin, the hair and/or the lips, **characterized in that** an emulsion according to any one of Claims 1 to 13 is applied to the skin, the hair and/or the lips.

16. Use of the emulsion according to any one of Claims 1 to 13, for the manufacture of a composition intended for caring for dry skin and/or dry lips and/or sensitive skin.
